(19) Europäisches Patentamt
European Patent Office
Office européen des brevets

(11) EP 4 732 835 A1

(12) EUROPEAN PATENT APPLICATION

(43) Date of publication:
**29.04.2026 Bulletin 2026/18**

(21) Application number: **24209123.9**

(22) Date of filing: **28.10.2024**

(51) International Patent Classification (IPC):
***A61K 31/277*** (2006.01) ***A61P 3/04*** (2006.01)

(52) Cooperative Patent Classification (CPC):
**A61P 3/04; A61K 31/277**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **CMR CureDiab Metabolic Research GmbH**
**40225 Düsseldorf (DE)**

(72) Inventors:
- **ECKEL, Jürgen**
  **40225 Düsseldorf (DE)**
- **ROHBECK, Elisabeth**
  **40225 Düsseldorf (DE)**

(74) Representative: **Eisenführ Speiser Patentanwälte Rechtsanwälte PartGmbB**
**Gollierstraße 4**
**80339 München (DE)**

# (54) THIOACRYLAMIDE DERIVATIVE FOR USE IN THE PREVENTION OR TREATMENT OF OBESITY

(57) The present invention relates to a thioacrylamide derivative for use in the prevention or treatment of obesity in a subject, to a pharmaceutical composition comprising such a thioacrylamide derivative for use in the prevention or treatment of obesity in a subject, and to the cosmetic, non-therapeutic use of a thioacrylamide derivative for reducing the body weight of a subject and/or for reducing or preventing body weight gain of a subject, and/or for reducing the body fat percentage of a subject.

EP 4 732 835 A1

## Description

The present invention relates to a thioacrylamide derivative for use in the prevention or treatment of obesity in a subject, to a pharmaceutical composition comprising such a thioacrylamide derivative for use in the prevention or treatment of obesity in a subject, and to the cosmetic, non-therapeutic use of a thioacrylamide derivative for reducing the body weight of a subject and/or for reducing or preventing body weight gain of a subject, and/or for reducing the body fat percentage of a subject.

In 2022, over 1 billion people were obese worldwide (879 million adults and 159 million children), representing more than a double of adult cases (and four times higher than cases among children) registered in 1990, wherein the obesity caused deaths are estimated to 2.8 million people per year.

Obesity is a medical condition, in which a subject has accumulated excessive body fat, such that potential risks for the subject's health arise. Obesity is a major cause of disability and is correlated with various diseases and conditions, particularly cardiovascular diseases, type 2 diabetes, liver diseases such as Metabolic dysfunction-associated steatotic liver disease (MASLD; formerly Nonalcoholic Fatty Liver Disease (NAFLD)) obstructive sleep apnoea, certain types of cancer, and osteoarthritis.

Cardiovascular diseases are typically caused or promoted by excessive fat levels in the blood, usually measured as elevated total triglycerides, total cholesterol, high density lipoprotein (HDL) cholesterol and low density lipoprotein (LDL) cholesterol, which accumulate and precipitate in the blood vessels of patients. Consequently, the vessel diameter available for blood transportation is reduced, which usually leads to increased blood pressure and a high risk of vessel damages.

Liver diseases are typically caused or promoted by excessive fat accumulation in the liver, which ultimately leads to liver damages. Such damages may be measured as elevated levels of Alanine transaminase (ALT) and aspartate aminotransferase (AST) in the blood of patients.

The World Health Organisation (WHO) defines obesity by the subject's body mass index (BMI) and its waist-to-hip ratio (WHR). Further, the BMI is used to classify certain classes of obesity.

According to the WHO's definition a subject suffers from obesity, in case its BMI is 30 $kg/m^2$ or higher and its WHR is 0.85 or higher (female subjects) or, respectively, 0.90 or higher (male subjects).

Further, the body fat percentage is another factor for defining obesity. Several studies have been performed to define obesity according to the body fat percentage, for example Potter et al., "Defining Overweight and Obesity by Percent Body Fat instead of Body Mass Index", J Clin Endocrinol Metab. 2024 May 15. As a conclusion, the studies arrive at similar definitions, i.e. a subject having a body fat percentage of 30 % or higher (male and female) are considered obese.

Different causes of obesity have been identified, including overeating, genetic mutations, often including mutations in the leptin-melanocortin system, further genetic diseases leading to obesity, such as Prader-Willi syndrome, Kleefstra syndrome (9q34.3 deletion syndrome), Börjeson-Forssman-Lehmann syndrome, or Carpenter syndrome, but also liver disorders or particular medication leading to the accumulation of body fat, usually by the administration of corticosteroids such as cortisol or prednisolone.

The current treatment of obesity usually includes a change of diet (amount, frequency and quality) and increasing the amount of physical exercise to change from a caloric surplus state of the subject to a calorie deficit state, which then leads to a reduction of body fat and body weight.

Additionally, pharmaceutical interventions or even surgery to remove the excessive fat are treatment options. Pharmaceuticals for removing the excessive fat are often based on substances, which reduce the appetite and/or the resorption of nutrition, substances, which interfere with the body weight regulation.

In the case of surgery, only the symptoms (excessive body fat) are reduced, but not their cause. Thus, it is frequently observed that subjects cannot maintain the improved state and start gaining weight again. For such cases, some options include e.g. applying a gastric band to reduce the gastric volume and achieve an earlier sense of satiety.

Usually, diet, exercise and pharmaceutical interventions / surgery are combined to effectively lead to a sustainable weight loss. Nevertheless, by changing their diet and exercise, a subject usually needs to drastically change the lifestyle, which was followed over years or decades, to arrive at and maintain a non-obese body weight. In many cases, social or familial factors further complicate the subject's attempt to arrive at and maintain a non-obese body weight.

Further, in some cases, a subject has arrived at a state, in which neither exercise nor surgery is possible. In such a state, a change of diet may not be sufficient for losing weight in the required amount and may even be harmful to the subject. In such cases, pharmaceutical interventions is highly required.

Moreover, subjects at risk of gaining excessive body fat and thus with a risk of obesity, such as subjects with a corresponding genetic background or subjects receiving medication promoting body fat gain, only have limited treatment options for preventing obesity.

A limited number of pharmaceutical interventions exists for treating or preventing obesity in a subject. However, the drawback of many of such pharmaceuticals is that the reduction of body weight is not limited to losing fat mass, but often leads to a reduction of lean mass (i.e. the fat-free mass of a subject, i.e. muscles, bones, etc.) as well, which is not desired

and may even lead to further complications in the subject's motor system.

As described above, the pharmaceutical options are a necessary (sometimes even the major) component of effective weight loss to treat obesity. However, as also described above, the options for treatment / prevention are rather limited and often come with the drawback of reducing lean mass as well.

In addition to the pharmacological treatment / prevention of obesity, there is also a large interest of the cosmetics industry in possibilities for reducing body weight or, respectively, excessive body fat. Several cosmetic options exist for reducing the body weight or, respectively, excessive body fat in subjects, which do not suffer from obesity or overweight but who wish to reduce or at least maintain their body weight or, respectively, body fat for cosmetic reasons. Usually these treatments are directed to local fat deposits and include approaches such as a liposuction or an injection lipolysis. These treatments are typically performed for aesthetic reasons only. Further, since these approaches are typically directed to local fat deposits, the effect on the overall body weight and/or body fat percentage is limited.

To address the overall body weight and/or body fat percentage, the medication for removing the excessive fat, as described above, may be applied. However, as also described above, the current options typically lead to a reduction of lean mass as well, which is not desired.

In light of the high number of affected subjects there is a great need to provide further options for treatment / prevention of obesity, preferably without the disadvantages described above. Further, there is also a need for cosmetics industry to provide an option for cosmetically reducing or maintaining the body weight of a subject.

Therefore, the primary object of the present invention was to provide a possibility for treating or preventing obesity in a subject, preferably without the disadvantages described above, and/or for reducing or maintaining the body weight of a subject.

The primary object of the present invention is solved by a thioacrylamide derivative for use in the prevention or treatment of obesity in a subject,
wherein the thioacrylamide derivative is selected from the group consisting of 2-Cyano-3-cyclopropyl-N-(4-fluoro-3-methyl-phenyl)-3-hydroxy-thioacrylamide (i.e. HK1), 2-Cyano-3-cyclopropyl-3-hydroxy-N-(3-methyl-4-nitro-phenyl)-thioacrylamide (i.e. HK3), a tautomer of HK1, a tautomer of HK3, or mixtures thereof, or pharmaceutically acceptable salts thereof or solvates thereof.

It was surprisingly found that the thioacrylamide derivative according to the present invention is able to reduce the body weight and the body weight gain. However, in contrast to currently available pharmaceutical options, the thioacrylamide derivative according to the present invention targeted the fat mass only and did not lead to a change in the lean mass.

It was particularly surprising in light of WO 2024/114970 A1, which describes said thioacrylamide derivative according to the present invention for use in the treatment of liver fibrosis caused by or resulting from non-alcoholic fatty liver disease (NAFLD). It was found in WO 2024/114970 A1 that the treatment with said thioacrylamide derivative according to the present invention did not lead to a change in the body weight or body weight gain in the tested mice.

The mice tested in WO 2024/114970 A1 received a standard diet and were thus not on a caloric surplus or even suffered from obesity. However, as shown in the below example section, an effect on the body weight and body weight gain was observed in mice receiving a high caloric diet. This effect was particularly surprising in light of WO 2024/114970 A1.

HK1 and HK3 belong to a family of positive allosteric modulators (PAMs) of the $\gamma$ aminobutyric acid type A (GABAA) receptor with exclusive peripheral action.

Preferably, HK1 refers to the compound according to the following structure:

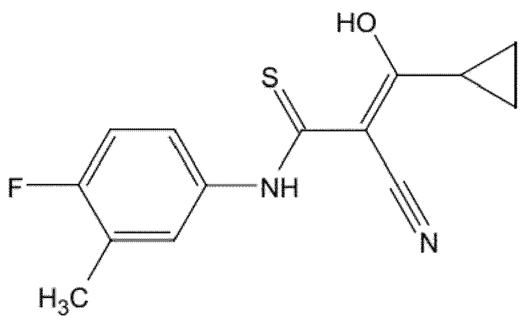

Preferably, HK3 refers to the compound according to the following structure:

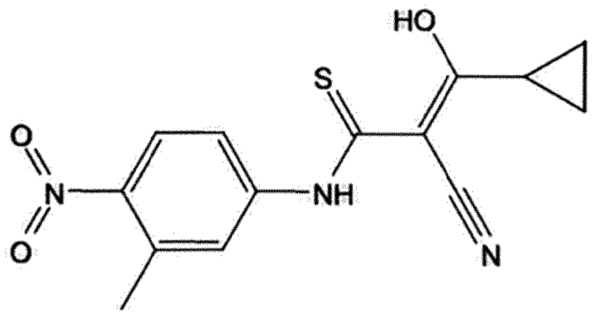

The term "tautomer", as used herein, preferably refers to the keto-enol-tautomer of a structure. Thus, preferably, the term "tautomer of HK1", as used herein, refers to the structure of HK1, as shown above, wherein the C=C-OH group next to the cyclopropyl group is a C-C=O group. Likewise, the term "tautomer of HK3", as used herein, preferably to the structure of HK3, as shown above, wherein the C=C-OH group next to the cyclopropyl group is a C-C=O group.

Preferably, the term "pharmaceutically acceptable salt", as used herein, refers to non-toxic salts. Preferably, the salts are derived from acids such as acetic acid, aconitic acid, citric acid, embolic acid, enanthic acid, hydrobromic acid, hydrochloric acid, lactic acid, maleic acid, malic acid, methanesulphonic acid, phosphoric acid, phthalic acid, salicylic acid, sorbic acid, succinic acid, sulfuric acid or tartaric acid.

Preferably, the term "solvate", as used herein, refers to a compound or salt thereof, further including a stoichiometric or non-stoichiometric amount of solvent bound by non-covalent intermolecular forces, e.g. a hydrate in case the solvent is water.

Preferably, the thioacrylamide derivative is or comprises 2-Cyano-3-cyclopropyl-3-hydroxy-N-(3-methyl-4-nitro-phenyl)-thioacrylamide (i.e. HK3) or a pharmaceutically acceptable salt thereof. Preferably, the thioacrylamide derivative is or comprises 2-Cyano-3-cyclopropyl-3-hydroxy-N-(3-methyl-4-nitro-phenyl)-thioacrylamide (i.e. HK3).

The term "obesity", as used herein, refers to a medical condition in which a subject has gained excessive body fat and thus extreme overweight.

Preferably, the term "obesity" is characterized or includes a body mass index (BMI) of 30 kg/m$^2$ or higher. Preferably, the body mass index is calculated by dividing the body weight m (kg) by the square body length l from head to toes (m$^2$):

$$BMI = \text{body weight} / (\text{body length})^2 = m / l^2 = kg / m^2$$

Preferably, the BMI provides the following classifications as described in the SuRF Report 2 of the WHO: underweight (under 18.5 kg/m$^2$), normal weight (18.5 to 24.9 kg/m$^2$), overweight (25 to 29.9 kg/m$^2$), and obese (30 kg/m$^2$or more).

Additionally or alternatively, it is preferred that the term "obesity" is characterized or includes a waist-to-hip ratio of 0.85 or higher (female subjects) or, respectively, 0.90 or higher (male subjects). Preferably, the waist-to-hip ratio is calculated by measuring the smallest circumference of the waist of a subject and the wides circumference of the hip. The resulting ratio of waist circumference to hip circumference is thus the waist-to-hip ratio and can be calculated e.g. as described by the University of Maryland on https://www.healthcalcu-lators.org/calculators/waist_hip.asp.

Additionally or alternatively, it is preferred that the term "obesity" is characterized or includes a body fat percentage of 30 % or higher (male and female). Preferably, the body fat percentage is measured by at least one method selected from dual energy X-ray absorptiometry, body average density measurement (total mass divided by total volume) including the Brozek formula or Siri formula, bioelectrical impedance analysis, near-infrared interactance, whole-body air displacement plethysmography, and underwater weighing, particularly preferably by a method comprising or being dual energy X-ray absorptiometry. Even further preferably the body fat percentage is measured by a method comprising or being MRI imaging, determining the proportion of the adipose tissue in the MRI images and applying the proportion to the total body weight to retrieve the fat mass.

Preferably, the subject is a human or animal, particularly preferably the subject is a human.

Preferably, the subject suffers from obesity or is expected to suffer from obesity. Preferably, the treatment of obesity is directed to a subject suffering from obesity. Preferably, the prevention of obesity is directed to a subject not (e.g. not yet) suffering from obesity. Preferably, the prevention of obesity is directed to a subject suffering from overweight (preferably with a body mass index of 25 to 29.9 kg/m$^2$). Preferably, the prevention of obesity is directed to a subject suffering from a liver disorder, such as metabolic-associated steatohepatitis (MASH). Preferably, the prevention of obesity

is directed to a subject suffering from a genetic mutation promoting an increase of the body weight, particularly an increase of the body fat percentage, such as a mutation in the leptin-melanocortin system. Preferably, the prevention of obesity is directed to a subject suffering from a genetic disorder, preferably Prader-Willi syndrome, preferably Kleefstra syndrome (9q34.3 deletion syndrome), preferably Börjeson-Forssman-Lehmann syndrome, preferably Carpenter syndrome. Preferably, the prevention of obesity is directed to a subject taking a pharmaceutical promoting an increase of the body weight, particularly an increase of the body fat percentage, preferably an antipsychotic (e.g. Clozapine, Haloperidol or Olanzapine), preferably an antidepressant (e.g. Tranylcypromine, Citalopram or Amitriptyline), preferably a beta blocker (e.g. metoprolol), preferably a corticosteroid (e.g. cortisol or prednisolone).

It is preferred for the present invention that the obesity is caused by or characterized by or includes at least one factor selected from the group consisting of

- overeating;

- lack of physical exercise;

- liver disorders, such as metabolic-associated steatohepatitis (MASH);

- genetic mutations, such as mutations in the leptin-melanocortin system; genetic disorders, such as Prader-Willi syndrome, Kleefstra syndrome (9q34.3 deletion syndrome), Börjeson-Forssman-Lehmann syndrome, or Carpenter syndrome;

- pharmaceuticals, such as antipsychotics (e.g. Clozapine, Haloperidol or Olanzapine), antidepressants (e.g. Tranylcypromine, Citalopram or Amitriptyline), beta blockers (e.g. metoprolol) or corticosteroids (e.g. cortisol or prednisolone);

- sleep deprivation;

- mental stress.

It is preferred that the treatment or prevention is characterized by or includes a reduction of the body weight or a reduction or prevention of body weight gain or a reduction of the body fat percentage. Particularly preferably, the treatment is characterized by or includes a reduction of the body weight. Particularly preferably, the treatment is characterized by or includes a reduction of the body fat percentage. Particularly preferably, the prevention is characterized by or includes a reduction or prevention of body weight gain.

Preferably, the term "increase of the body weight" and "body weight gain", as used herein, are used interchangeably and refer to or include an increase of the body weight over a defined time period, preferably one month, of at least 5 %, preferably at least 7.5 %, particularly at least 10 %, further preferably at least 12.5 %, more preferably at least 15 %, especially preferably at least 17.5 % even further preferably at least 20 %, compared to and based on the previous time period of the same duration. Preferably, the increase or reduction is determined by comparing the average value within the defined time period with the average value within the previous time period of the same duration. E.g. a subject has an average body weight of 75 kg over 1 month. An increase of the body weight of at least 5 % would then refer to an average body weight of 78,75 kg in the subsequent month. Preferably, the defined time period is 1 month. Preferably, the defined time period is 2 months. Preferably, the defined time period is 3 months. Preferably, the defined time period is 4 months. Preferably, the defined time period is 6 months. Preferably, the defined time period is 12 months. The same applies accordingly to the term "increase of the body fat percentage".

Preferably, the term "reduction of the body weight" and "body weight loss", as used herein, are used interchangeably and refer to or include a reduction of the body weight over a defined time period, preferably one month, of at least 5 %, preferably at least 7.5 %, particularly at least 10 %, further preferably at least 12.5 %, more preferably at least 15 %, especially preferably at least 17.5 % even further preferably at least 20 %, compared to and based on the previous time period of the same duration. Preferably, the increase or reduction is determined by comparing the average value within the defined time period with the average value within the previous time period of the same duration. E.g. a subject has an average body weight of 75 kg over 1 month. A reduction of the body weight of at least 5 % would then refer to an average body weight of 71,25 kg in the subsequent month. Preferably, the defined time period is 1 month. Preferably, the defined time period is 2 months. Preferably, the defined time period is 3 months. Preferably, the defined time period is 4 months. Preferably, the defined time period is 6 months. Preferably, the defined time period is 12 months. The same applies accordingly to the term "reduction of the body fat percentage".

Preferably, the term "reduction of body weight gain", as used herein refers to a reduction over a defined time period, preferably one month, of at least 5 %, preferably at least 7.5 %, particularly at least 10 %, further preferably at least

12.5 %, more preferably at least 15 %, especially preferably at least 17.5 % even further preferably at least 20 %, compared to and based on the previous time period of the same duration. Preferably, the increase or reduction is determined by comparing the average value within the defined time period with the average value within the previous time period of the same duration. E.g. a subject has a body weight gain of 2 kg over 1 month, comparing the body weight at the beginning of the month with the body weight at the end of the month. A reduction of at least 5 % would then refer to a body weight gain of at most 1.9 kg in the subsequent month. Preferably, the defined time period is 1 month. Preferably, the defined time period is 2 months. Preferably, the defined time period is 3 months. Preferably, the defined time period is 4 months. Preferably, the defined time period is 6 months. Preferably, the defined time period is 12 months.

Preferably, the term "prevention of body weight gain", as used herein refers to an increase or a reduction over a defined time period, preferably one month, of less than 20 %, preferably less than 17.5 %, particularly less than 15 %, further preferably less than 12.5 %, more preferably less than 10 %, especially preferably less than 7.5 % even further preferably less than 5 %, compared to and based on the previous time period of the same duration. Preferably, the increase or reduction is determined by comparing the average value within the defined time period with the average value within the previous time period of the same duration. E.g. a subject has an average body weight of 75 kg over 1 month. An increase of the body weight of less than 5 % would then refer to an average body weight of less than 78,75 kg in the subsequent month. Preferably, the defined time period is 1 month. Preferably, the defined time period is 2 months. Preferably, the defined time period is 3 months. Preferably, the defined time period is 4 months. Preferably, the defined time period is 6 months. Preferably, the defined time period is 12 months

Preferably, a month refers to a calendar month. Preferably, a month refers to a time period of 30 days.

Preferably, the body weight or, respectively, body weight gain refers to the adipose tissue.

It has been found that already low amounts of the thioacrylamide derivative are sufficient for the prevention or treatment of obesity, as described herein. It is thus preferred for the thioacrylamide derivative for use according to the invention that the prevention or treatment comprises the administration of at least 1 mg/kg body weight of the subject, preferably at least 2.5 mg/kg, preferably at least 5 mg/kg, preferably at least 7.5 mg/kg, preferably at least 10 mg/kg, of the thioacrylamide derivative.

It is further preferred for the thioacrylamide derivative for use according to the invention that the prevention or treatment comprises the administration of a daily dose of at least 1 mg/kg body weight of the subject, preferably at least 2.5 mg/kg, preferably at least 5 mg/kg, preferably at least 7.5 mg/kg, preferably at least 10 mg/kg, of the thioacrylamide derivative.

Preferably, the term "daily dose", as used herein, refers to the total dose of the thioacrylamide derivative(s) selected from the group consisting of 2-Cyano-3-cyclopropyl-N-(4-fluoro-3-methyl-phenyl)-3-hydroxy-thioacrylamide (HK1), 2-Cyano-3-cyclopropyl-3-hydroxy-N-(3-methyl-4-nitro-phenyl)-thioacrylamide (HK3), a tautomer of HK1, a tautomer of HK3, or mixtures thereof, or pharmaceutically acceptable salts thereof or solvates thereof.

Further preferably, the term "daily dose", as used herein, refers to an average daily amount per body weight of the thioacrylamide derivative selected from the group consisting of 2-Cyano-3-cyclopropyl-N-(4-fluoro-3-methyl-phenyl)-3-hydroxy-thioacrylamide (HK1), 2-Cyano-3-cyclopropyl-3-hydroxy-N-(3-methyl-4-nitro-phenyl)-thioacrylamide (HK3), a tautomer of HK1, a tautomer of HK3, or mixtures thereof, or pharmaceutically acceptable salts thereof or solvates thereof, which is administered to a subject and does not necessarily require the administration to be performed every single day of a given time period. If the administration of the thioacrylamide derivative(s) is performed once or more in a given number of days, the daily dose can be calculated by dividing the total administered amount of the thioacrylamide derivative(s) per body weight of the subject by the respective given number of days. Preferably, the term "daily dose of a mixture" refers to the summed dose of the respective components of the mixture per day.

It is further preferred for the thioacrylamide derivative or pharmaceutical composition for use according to the invention that the administration is performed at an administration scheme of at least once in 7 days, preferably at least once in 6 days, preferably at least once in 5 days, preferably at least once in 4 days, preferably at least once in 3 days, preferably at least once in 2 days, preferably at least once a day, preferably at least twice a day, preferably at least three times a day, preferably at least four times a day, preferably at least five times a day.

Preferably, the administration scheme is applied over a time period of at least 1 week, preferably at least 2 weeks, preferably at least 3 weeks, preferably at least 4 weeks, preferably at least 6 weeks, preferably at least 2 months, preferably at least 3 months, preferably at least 4 months, preferably at least 5 months, preferably at least 6 months, preferably at least 7 months, preferably at least 8 months, preferably at least 9 months, preferably at least 10 months, preferably at least 11 months, preferably at least 12 months, preferably at least 15 months, preferably at least 18 months, preferably at least 24 months.

Applying the administration scheme for a certain time period as described herein and within a certain regularity as described herein are not mutually exclusive. E.g., an administration of at least once in 7 days for at least 4 weeks describes the regularity of once in 7 days for a duration of at least 4 weeks or longer. The term "once in 7 days", as used herein, is to be understood as one administration within a time frame of 7 days, wherein the administration may be at the beginning, the end or at any other time point within the 7 days. In case the administration is repeated, i.e. the time period of the

administration scheme is longer than the administration interval, the time point of the respective administration (the beginning, the end or at any other time point within the interval) may vary between the respective administration intervals. As an example, in an administration of at least once in 7 days for at least 4 weeks, the administration in the first 7 days (first interval) may be performed at the beginning (day 1) of the interval and the administration in the second 7 days (second interval) may be performed on day 3 of the interval. The same applies accordingly to any other regularity and time period as described herein.

Preferably, the administration is performed by or comprises an administration via an administration route selected from the group consisting of aural, buccal, endobronchial, enteral, inhalative, intraarterial, intraarticular, intrabronchial, intraductal, intragastric, intragluteal, intracardiac, intracavernous, intracutaneous, intralesional, intralumbar, intralymphatic, intramammary, intramuscular, intranasal, intraneural, intraocular, intraosseous, intraperitoneal, intrapleural, intrapulmonary, intrathecal, intratracheal, intraurethral, intrauterine, intravenous, intraventricular, intravitreal, conjunctival, nasal, oral, parenteral, peroral, perineural, rectal, retrobulbar, subcutaneous, sublingual, transdermal, vaginal, and absorption over a mucous membrane.

The present invention further relates to a pharmaceutical composition comprising a thioacrylamide derivative according to the invention for use in the prevention or treatment of obesity, as described and defined herein.

What was said herein with regard to the definitions and preferred features of e.g. the thioacrylamide derivative, the treatment and/or prevention, of obesity, of the subject, of the administration of the thioacrylamide derivative, applies accordingly to the pharmaceutical composition according to the invention.

Particularly, all features described for the thioacrylamide derivative for use according to the invention apply accordingly to the pharmaceutical composition for use according to the invention.

Preferably, the dosage form of a pharmaceutical composition is selected from the group consisting of solid dosage forms such as tablets, sublingual tablets, melting tablets, dragees, capsules, soft capsules, hard capsules, gastroresistant capsules, granules, powders and printlets, semi-solid dosage forms such as ointments, creams, pastes and gels, liquid dosage forms such as suspensions, crystal suspensions, emulsions, solutions, drops, nasal drops, eye drops, ear drops, syrups, juices, injection solutions, injection dispersions, injection solutions and injection solution concentrates, and further dosage forms such as suppositories, lozenges, effervescent tablets, effervescent powders, inhalation powders, plasters, metered dose inhalers, foams, sprays, sublingual sprays, shampoos, mouthwashes, vaginal suppositories, vaginal tablets and vaginal capsules.

It is preferred for the pharmaceutical composition according to the invention that the composition comprises the thioacrylamide derivative in a range of from 0.0001 wt.-% to 20 wt.-%, preferably of from 0.0005 to 15 wt.-%, particularly preferably of from 0.001 to 12.5 wt.-%, further preferably of from 0.005 to 10 wt.-%, even further preferably of from 0.01 to 7.5 wt.-%, especially preferably of from 0.05 to 5 wt.-%, more preferably of from 0.1 to 2.5 wt.-%, even more preferably of from 0.5 to 1.5 wt.-%, based on the total weight of the pharmaceutical composition.

In case the thioacrylamide derivative refers to a mixture of substances as described herein, the indicated wt.-% preferably refers to the summed value of the single substances in said mixture.

Preferably, the thioacrylamide derivative in the pharmaceutical composition is or comprises 2-Cyano-3-cyclopropyl-3-hydroxy-N-(3-methyl-4-nitro-phenyl)-thioacrylamide (i.e. HK3) or a pharmaceutically acceptable salt thereof. Preferably, the thioacrylamide derivative in the pharmaceutical composition is or comprises 2-Cyano-3-cyclopropyl-3-hydroxy-N-(3-methyl-4-nitro-phenyl)-thioacrylamide (i.e. HK3).

Preferably, the pharmaceutical composition according to the invention further comprises one or more substances selected from carriers, excipients, stabilizers, binders, fillers, disintegrants, and lubricants.

The present invention further relates to the cosmetic, non-therapeutic use of a thioacrylamide derivative or a pharmaceutical composition comprising a thioacrylamide derivative as defined herein,

for reducing the body weight of a subject and/or for reducing or preventing body weight gain of a subject, and/or for reducing the body fat percentage of a subject,

wherein the thioacrylamide derivative is selected from the group consisting of 2-Cyano-3-cyclopropyl-N-(4-fluoro-3-methyl-phenyl)-3-hydroxy-thioacrylamide (i.e. HK1), 2-Cyano-3-cyclopropyl-3-hydroxy-N-(3-methyl-4-nitro-phenyl)-thioacrylamide (i.e. HK3), a tautomer of HK1, a tautomer of HK3, or mixtures thereof, or pharmaceutically acceptable salts thereof or solvates thereof,

preferably wherein the subject is in a caloric surplus state.

What was said herein with regard to the definitions and preferred features of e.g. the thioacrylamide derivative, the treatment and/or prevention, of obesity, of the subject, of the administration of the thioacrylamide derivative, the pharmaceutical composition according to the invention, applies accordingly to the use according to the invention if not stated otherwise.

Particularly, all features described for the thioacrylamide derivative for use according to the invention and the pharmaceutical composition for use according to the invention apply accordingly to the use according to the invention if not stated otherwise.

The cosmetic, non-therapeutic use differs from the above treatment and prevention of obesity as a medical condition by that a different group of subjects is addressed. The subjects addressed by the cosmetic use are subjects not suffering from obesity or overweight, each as defined above.

Thus, the subjects addressed by the cosmetic use are preferably subjects having a body mass index of less than 25 kg/$m^2$, preferably less than 22.5 kg/$m^2$, particularly preferably less than 20 kg/$m^2$. The subjects addressed by the cosmetic use are preferably subjects having a waist-to-hip ratio of less than 0.80, preferably of less than 0.75, particularly preferably of less than 0.70. The subjects addressed by the cosmetic use are preferably subjects having a body fat percentage of less than 25 %, preferably less than 22.5 %, particularly preferably less than 20 %.

The term "reducing the body weight", as used herein, corresponds to the term "reduction of the body weight", as defined herein. The term "reducing body weight gain", as used herein, corresponds to the term "reduction of body weight gain", as defined herein. The term "preventing body weight gain", as used herein, corresponds to the term "prevention of body weight gain", as defined herein. The term "reducing the body fat percentage", as used herein, corresponds to the term "reduction of the body fat percentage", as defined herein.

The term "caloric surplus state", as used herein, preferably refers to a state, in which the consumed calories exceed the calories burnt by a subject. Preferably, the caloric state may be determined by calculating the required calories for maintaining the current body weight and comparing the value with the consumed calories.

For calculating the calories required for maintaining the current body weight, several tools are available, such as https://www.esn.com/en/paqes/caloriecalculator. Preferably, the calories required for maintaining the current body weight are determined with the calculator provided by https://www.esn.com/en/paqes/caloriecalculator.

The calories required for maintaining the current body weight correspond to the calories burnt by the subject (in this case the consumed/required calories equal the burnt calories such that the body weight is maintained). In case the consumed calories exceed the calories required for maintaining the current body weight, the body weight increases. In case the consumed calories are lower than the calories required for maintaining the current body weight, the body weight is reduced. Preferably, the term "caloric surplus state", as used herein, preferably refers to a state, in which the consumed calories exceed the calories burnt by a subject by at least 5 %, preferably at least 10 %, further preferably at least 15 %, particularly preferably at least 20 %, especially preferably at least 25 %, based on the calories burnt by a subject.

Thus, the term "caloric surplus state", as used herein, preferably refers to a state, in which the subject's body weight increased in average by at least 0.5 %, preferably at least 0.75 %, further preferably at least 1 %, particularly preferably at least 1.25 %, especially preferably at least 1.5 % per month, based on the body weight of the beginning of the month, for at least 2 subsequent months, preferably at least 3 subsequent months, further preferably at least 4 subsequent months, particularly preferably at least 5 subsequent months, especially preferably at least 6 subsequent months, more preferably at least 12 subsequent months.

For example, an increase in average by 1 % per month over 3 months may comprise an increase by 1 % in month 1, an increase in 0.75 % in month 2 and an increase by 1.5 % in month 3, which corresponds to an average increase of 1.083 %.

Similarly, an increase in average by e.g. 1 % may also comprise a reduction in one or more months, provided that the average increase over the defined duration is e.g. 1%.

It is preferred in the use according to the invention that the thioacrylamide derivative is or comprises 2-Cyano-3-cyclopropyl-3-hydroxy-N-(3-methyl-4-nitro-phenyl)-thioacrylamide (i.e. HK3) or a pharmaceutically acceptable salt thereof. It is preferred in the use according to the invention that the thioacrylamide derivative is or comprises 2-Cyano-3-cyclopropyl-3-hydroxy-N-(3-methyl-4-nitro-phenyl)-thioacrylamide (i.e. HK3).

It is preferred that the use according to the invention comprises administering at least 5 mg/kg per body weight of the subject, preferably at least 7.5 mg/kg per body weight of the subject, preferably at least 10 mg/kg per body weight of the subject, of the thioacrylamide derivative.

It is preferred that the use according to the invention comprises the administration of a daily dose of at least 1 mg/kg body weight of the subject, preferably at least 2.5 mg/kg, preferably at least 5 mg/kg, preferably at least 7.5 mg/kg, preferably at least 10 mg/kg, of the thioacrylamide derivative, as defined herein.

It is preferred in the use according to the invention that the body weight or, respectively, body weight gain may refer to the adipose tissue.

What was said herein with regard to the administration doses or route applies accordingly to the use according to the invention.

The present invention further relates to a method for treating or preventing obesity in a subject, the method comprising

- administering to a subject in need thereof a thioacrylamide derivative,

wherein the thioacrylamide derivative is selected from the group consisting of 2-Cyano-3-cyclopropyl-N-(4-fluoro-3-methyl-phenyl)-3-hydroxy-thioacrylamide (i.e. HK1), 2-Cyano-3-cyclopropyl-3-hydroxy-N-(3-methyl-4-nitro-phenyl)-thioacrylamide (i.e. HK3), a tautomer of HK1, a tautomer of HK3, or mixtures thereof, or pharmaceutically acceptable salts thereof or solvates thereof.

Preferably, the thioacrylamide derivative is or comprises 2-Cyano-3-cyclopropyl-3-hydroxy-N-(3-methyl-4-nitro-phenyl)-thioacrylamide (i.e. HK3) or a pharmaceutically acceptable salt thereof. Preferably, the thioacrylamide derivative is or comprises 2-Cyano-3-cyclopropyl-3-hydroxy-N-(3-methyl-4-nitro-phenyl)-thioacrylamide (i.e. HK3).

For example, the thioacrylamide derivative may be administered in form of a pharmaceutical composition as described herein.

What was said herein with regard to the definitions and preferred features of e.g. the thioacrylamide derivative, the treatment and/or prevention, of obesity, of the subject, of the administration of the thioacrylamide derivative, the pharmaceutical composition according to the invention, applies accordingly to the use according to the invention if not stated otherwise.

For example, the obesity may be caused by or characterized by or includes at least one factor selected from the group consisting of

- overeating;
- lack of physical exercise;
- liver disorders, such as metabolic-associated steatohepatitis (MASH);
- genetic mutations, such as mutations in the leptin-melanocortin system; genetic disorders, such as Prader-Willi syndrome, Kleefstra syndrome (9q34.3 deletion syndrome), Börjeson-Forssman-Lehmann syndrome, or Carpenter syndrome;
- pharmaceuticals, such as antipsychotics (e.g. Clozapine, Haloperidol or Olanzapine), antidepressants (e.g. Tranylcypromine, Citalopram or Amitriptyline), beta blockers (e.g. metoprolol) or corticosteroids (e.g. cortisol or prednisolone);
- sleep deprivation;
- mental stress.

For example, the treatment or prevention in the method may be characterized by or may include a reduction of the body weight or a reduction or prevention of body weight gain.

For example, the body weight or, respectively, body weight gain may refer to the adipose tissue.

For example, the method comprises the administration of at least 5 mg/kg per body weight of the subject, preferably at least 7.5 mg/kg per body weight of the subject, preferably at least 10 mg/kg per body weight of the subject, of the thioacrylamide derivative.

The present invention further relates to a method for reducing the body weight of a subject and/or for reducing or preventing body weight gain of a subject, and/or for reducing the body fat percentage of a subject, the method comprising

- administering to a subject in need thereof a thioacrylamide derivative,

wherein the thioacrylamide derivative is selected from the group consisting of 2-Cyano-3-cyclopropyl-N-(4-fluoro-3-methyl-phenyl)-3-hydroxy-thioacrylamide (i.e. HK1), 2-Cyano-3-cyclopropyl-3-hydroxy-N-(3-methyl-4-nitro-phenyl)-thioacrylamide (i.e. HK3), a tautomer of HK1, a tautomer of HK3, or mixtures thereof, or pharmaceutically acceptable salts thereof or solvates thereof

preferably wherein the subject is in a caloric surplus state.

Preferably, the thioacrylamide derivative is or comprises 2-Cyano-3-cyclopropyl-3-hydroxy-N-(3-methyl-4-nitro-phenyl)-thioacrylamide (i.e. HK3) or a pharmaceutically acceptable salt thereof. Preferably, the thioacrylamide derivative is or comprises 2-Cyano-3-cyclopropyl-3-hydroxy-N-(3-methyl-4-nitro-phenyl)-thioacrylamide (i.e. HK3).

For example, the thioacrylamide derivative may be administered in form of a pharmaceutical composition as described herein.

What was said herein with regard to the definitions and preferred features of e.g. the thioacrylamide derivative, the treatment and/or prevention, of obesity, of the subject, of the administration of the thioacrylamide derivative, the pharmaceutical composition according to the invention, applies accordingly to the use according to the invention if not stated otherwise.
For example, the body weight or, respectively, body weight gain may refer to the adipose tissue.
For example, the method comprises the administration of at least 5 mg/kg per body weight of the subject, preferably at least 7.5 mg/kg per body weight of the subject, preferably at least 10 mg/kg per body weight of the subject, of the thioacrylamide derivative.

Fig. 1 shows the results of Example 1, wherein the measured body weight is shown for mice receiving a high fat diet (DIO) and the vehicle, i.e. 0.5% carboxymethyl cellulose and 1% Tween 20 in water, (control; black circles, DIO+vehicle) and for mice receiving a high fat diet (DIO) and the thioacrylamide derivative HK3 (treatment; white circles, DIO+HK3), with ####: $p<0.0001$ with a One-Way ANOVA with Tukey post test, n = 6-7.

Fig. 2 shows the results of Example 1, wherein the measured body weight gain is shown for mice receiving a high fat diet (DIO) and the vehicle, i.e. 0.5% carboxymethyl cellulose and 1% Tween 20 in water, (control; black circles, DIO+vehicle) and for mice receiving a high fat diet (DIO) and the thioacrylamide derivative HK3 (treatment; white circles, DIO+HK3), with ####: $p<0.0001$ with a One-Way ANOVA with Tukey post test, n = 6-7.

Fig. 3 shows the results of Example 1, wherein the measured food intake is shown for mice receiving a high fat diet (DIO) and the vehicle, i.e. 0.5% carboxymethyl cellulose and 1% Tween 20 in water, (control; black boxes DIO+vehicle) and for mice receiving a high fat diet (DIO) and the thioacrylamide derivative HK3 (treatment; white boxes DIO+HK3), wherein no significant differences were observed.

Fig. 4 shows the results of Example 1, wherein the measured fat mass, lean mass, total body weight, as well as fat-to-lean mass ratio is shown for mice receiving a high fat diet (DIO) and the vehicle, i.e. 0.5% carboxymethyl cellulose and 1% Tween 20 in water, (control; black bar; DIO+vehicle) and for mice receiving a high fat diet (DIO) and the thioacrylamide derivative HK3 (treatment; white bar; DIO+HK3), with **: $p<0.01$; unpaired student t-test; n=6-7.

Fig. 5 shows the results of Example 1, wherein the blood chemistry biomarker analysis regarding Alanine transaminase (ALT) and aspartate aminotransferase (AST) is shown for mice receiving a normal diet (Normal diet; grey bar), mice receiving a high fat diet and the vehicle, i.e. 0.5% carboxymethyl cellulose and 1% Tween 20 in water, (control; black bar; DIO+Vehicle) and for mice receiving a high fat diet (DIO) and the thioacrylamide derivative HK3 (treatment; white bar; DIO+HK3), with *: $p<0.05$; **: $p<0.01$ with a One-Way ANOVA with Tukey post test, n = 3/6/7.

Fig. 6 shows the results of Example 1, wherein the blood chemistry biomarker analysis regarding total trigycerides (TG), total cholesterol (TC), HDL-cholesterol (HDL-C) and LDL-cholesterol (LDL-C) is shown for mice receiving a normal diet (Control), mice receiving a high fat diet and the vehicle, i.e. 0.5% carboxymethyl cellulose and 1% Tween 20 in water, (DIO+vehicle) and for mice receiving a high fat diet and the thioacrylamide derivative HK3 (DIO+HK3), with ns: not significant; *: $p<0.05$; **: $p<0.01$; ***: $p<0.001$; ****: $p<0.0001$ with a One-Way ANOVA with Tukey post test, n = 3/6/7.

Further aspects and advantages of the invention result from the subsequent description of preferred examples.

## Examples

### Example 1: Diet-induced obesity (DIO) mice

The objective of this study is to evaluate the *in vivo* therapeutic efficacy of HK3 on the DIO mouse model.
The number of animals in each group and details of the route of administration, dose and report are shown in the table below.

| Group | Animal No. | Treatment | Dosing level (mg/kg) | Dosing volume (mL/kg) | Route of Administration | Dosing period |
|---|---|---|---|---|---|---|
| 1 | 3 | Normal diet Control | NA | 10 | oral | Once a day, 30 days |
| 2 | 6 | DIO + Vehicle | NA | 10 | oral | Once a day, 30 days |

(continued)

| Group | Animal No. | Treatment | Dosing level (mg/kg) | Dosing volume (mL/kg) | Route of Administration | Dosing period |
|---|---|---|---|---|---|---|
| 3 | 7 | DIO + HK3 | 25 | 10 | oral | Once a day, 30 days |

***Animal information:***

| | |
|---|---|
| **Species** | *Mus musculus* |
| **Strain** | C57BL/6J |
| **Supplier** | Cyagen Biosciences Inc. |
| **Gender** | Male |
| **Average age of animals at study initiation** | 18 weeks (estimated average age at treatment) |
| **Housing level** | SPF |

***Animal Housing Condition:***

| | |
|---|---|
| **Cage type** | Polysulfone IVC cage (325mm × 210mm × 180mm) |
| **Housing density** | Up to 6 mice per cage |
| **Temperature** | 20 - 26°C |
| **Humidity** | 40 - 70% |
| **Light cycle** | 12 hours light and 12 hours dark |
| **Bedding material** | Crushed corncob bedding, autoclaved; changed weekly |

| | |
|---|---|
| **Water** | Autoclaved filtered RO (reverse osmosis) softened water, ad libitum |
| **Animal identification** | Ear tag. |
| **Animal welfare monitoring** | Daily cage side observations, weekly clinical observations |

The mice were acclimated for one week under standard rodent diet ad libitum until they reached six weeks of age, followed by 12 weeks on a high-fat diet (HFD) for Groups 2 and 3. Group 1 continued to receive the standard rodent diet.

As high-fat diet, a commercial feed with 60 % kcal % fat was fed (D12492; Research Diet Inc, US; Formulated by E. A. Ulman, Ph.D., Research Diets, Inc., 8/26/98 and 3/11/99.; https://info.taconic.com/hs-fs/hub/355513/file-2452871853-pdf/Technical Library/D12492.pdf):

| Ingredient | Amount [g] | kcal |
|---|---|---|
| Casein, 80 Mesh | 200 | 800 |
| L-Cystine | 3 | 12 |
| Maltodextrin 10 | 125 | 500 |
| Sucrose | 68.8 | 275.2 |
| Cellulose, BW200 | 50 | 0 |
| Soybean Oil | 25 | 225 |

(continued)

| Ingredient | Amount [g] | kcal |
|---|---|---|
| Lard (typical analysis of cholesterol in lard = 0.95 mg/gram) | 245 | 2205 |
| Mineral Mix, S10026 | 10 | 0 |
| DiCalcium Phosphate | 13 | 0 |
| Calcium Carbonate | 5.5 | 0 |
| Potassium Citrate, 1 H2O | 16.5 | 0 |
| Vitamin Mix, V10001 | 10 | 40 |
| Choline Bitartrate | 2 | 0 |
| FD&C Blue Dye #1 | 0.05 | 0 |
| **Total** | **773.85** | **4057** |
| Protein | 26.2 % | 20 % |
| Carbohydrates | 26.3 % | 20 % |
| Fat | 34.9 % | 60 % |

***Study initiation and treatment:***

After the 18 weeks described above, the study was initiated (day 0). Groups 1 and 2 received the vehicle (orally), Group 3 received 25 mg/kg HK3 (orally) once a day for 30 days (day 30). During the study, Group 1 continued to receive the standard rodent diet, Groups 2 and 3 continued to receive the high-fat diet (HFD).

As a vehicle, a mixture of 0.5% carboxymethyl cellulose and 1% Tween 20 (Polysorbate 20) in water was used. The vehicle was prepared by completely dissolving sodium carboxymethyl cellulose in autoclaved, pre-heated (app. 80°C) water, adding Tween 20, stirring until the carboxymethyl cellulose and the Tween are completely dissolved, adjusting the volume of vehicle solution, aliquoting and storing the vehicle solution at 5°C ±3°C until required (day of application).

Before administration, the vehicle formulation was adjusted to room temperature and thoroughly mixed until a homogenous, milky white emulsion was achieved.

For the administration of HK3, the vehicle was prepared and stored as described above. At the day of administration, aliquots of the stored vehicle solution were adjusted to room temperature. The required dose of HK3 powder was provided, added to the vehicle solution and thoroughly mixed until a homogenous, yellow solution was achieved.

***Observations and data collection:***

The body weight of all mice was recorded each day. The food intake was recorded weekly (Group 1) or twice a week (Groups 2 and 3).

At the end of the study the body composition of the mice was measured using an MRI device (NIUMAG, QMR06-090H) to assess the total body fat and the lean mass of each mouse. The proportion of the adipose tissue in the MRI images was determined and applied to the total body weight to retrieve the fat mass.

Further, blood was collected and serum was separated in all mice at the end of the study to analyse the blood chemistry biomarkers (TG, TC, HDL-C, LDL-C, ALT, AST).

Means ± standard error of the mean (SEM) are presented in the figures.

***Results:***

During the study, HK3 significantly reduced body weight in DIO mice ($p<0.0001$, Figure 1).

Further, the body weight gain in HK3-treated mice was markedly reduced compared to vehicle-treated animals ($p<0.0001$, Figure 2), whereas no significant differences in food intake (Figure 3) between the HK3-treated and vehicle-treated groups were observed.

After the 30-day treatment period, the total body weight of the vehicle-treated mice reached 48.7±0.8g, whereas the body weight of HK3-treated mice was significantly lower at 43.5±0.9g ($p<0.01$, Figure 4). Interestingly, the lean mass did not change by HK3-treatment (22.1±0.3g vs. 21.6±0.5g). This result is a great advantage over GLP-1 based drugs, which lead to reductions in lean mass, especially muscle mass (Neeland et al., 2024, Changes in lean body mass with

glucagon-like peptide-1-based therapies and mitigation strategies. In: Diabetes, obesity & metabolism 26 Suppl 4, S. 16-27. DOI: 10.1111/dom.15728).

In addition, blood chemistry biomarker analysis (Figures 5 and 6) underlines the beneficial effect of HK3. The biomarkers ALT (alanine transaminase) as well as AST (aspartate aminotransferase), representing liver damages / diseases, are elevated in the DIO mouse group, corresponding to the known damaging impact of obesity on the liver of subjects.

However, HK3 is able to reduce elevated ALT (alanine transaminase) as well as AST (aspartate aminotransferase) concentration in the DIO mouse group (203.0$\pm$23.53 vs. 289.2$\pm$12.97 and 81.34$\pm$15.18 vs 174.8$\pm$27.59, $p<0.01$, $p<0.05$, respectively; Figure 5).

Triglyceride levels are significantly reduced by 33%, and total cholesterol by 36% in presence of HK3 (Figure 6), which demonstrates a positive effect of HK3 on liver damages caused by obesity.

These results demonstrate that HK3 significantly inhibited body weight gain, providing a beneficial effect for the treatment / prevention of obesity and its suitability for cosmetically reducing the body weight, body weight gain and body fat percentage of a subject.

## Claims

1. Thioacrylamide derivative for use in the prevention or treatment of obesity in a subject,
wherein the thioacrylamide derivative is selected from the group consisting of 2-Cyano-3-cyclopropyl-N-(4-fluoro-3-methyl-phenyl)-3-hydroxy-thioacrylamide (i.e. HK1), 2-Cyano-3-cyclopropyl-3-hydroxy-N-(3-methyl-4-nitro-phenyl)-thioacrylamide (i.e. HK3), a tautomer of HK1, a tautomer of HK3, or mixtures thereof, or pharmaceutically acceptable salts thereof or solvates thereof.

2. Pharmaceutical composition comprising a thioacrylamide derivative as defined in claim 1 for use in the prevention or treatment of obesity.

3. Pharmaceutical composition comprising the thioacrylamide derivative in a range of from 0.0001 wt.-% to 20 wt.-%, based on the total weight of the pharmaceutical composition.

4. Thioacrylamide derivative or pharmaceutical composition for use according to one of the preceding claims, wherein the thioacrylamide derivative or the thioacrylamide derivative in the pharmaceutical composition is or comprises 2-Cyano-3-cyclopropyl-3-hydroxy-N-(3-methyl-4-nitro-phenyl)-thioacrylamide (i.e. HK3) or a pharmaceutically acceptable salt thereof.

5. Thioacrylamide derivative or pharmaceutical composition for use according to one of the preceding claims, wherein the obesity is caused by or **characterized by** or includes at least one factor selected from the group consisting of

   - overeating;
   - lack of physical exercise;
   - liver disorders, such as metabolic-associated steatohepatitis (MASH);
   - genetic mutations, such as mutations in the leptin-melanocortin system; genetic disorders, such as Prader-Willi syndrome, Kleefstra syndrome (9q34.3 deletion syndrome), Börjeson-Forssman-Lehmann syndrome, or Carpenter syndrome;
   - pharmaceuticals, such as antipsychotics (e.g. Clozapine, Haloperidol or Olanzapine), antidepressants (e.g. Tranylcypromine, Citalopram or Amitriptyline), beta blockers (e.g. metoprolol) or corticosteroids (e.g. cortisol or prednisolone);
   - sleep deprivation;
   - mental stress.

6. Thioacrylamide derivative or pharmaceutical composition for use according to one of the preceding claims, wherein the treatment or prevention is **characterized by** or includes a reduction of the body weight or a reduction or prevention of body weight gain.

7. Thioacrylamide derivative or pharmaceutical composition for use according to claim 6, wherein the body weight or, respectively, body weight gain refers to the adipose tissue.

8. Thioacrylamide derivative or pharmaceutical composition for use according to one of the preceding claims, wherein

the treatment or prevention comprises the administration of at least 5 mg/kg per body weight of the subject, preferably at least 7.5 mg/kg per body weight of the subject, preferably at least 10 mg/kg per body weight of the subject, of the thioacrylamide derivative.

**9.** Cosmetic, non-therapeutic use of a thioacrylamide derivative or a pharmaceutical composition comprising a thioacrylamide derivative as defined in one of the preceding claims

for reducing the body weight of a subject and/or for reducing or preventing body weight gain of a subject, and/or for reducing the body fat percentage of a subject,
wherein the thioacrylamide derivative is selected from the group consisting of 2-Cyano-3-cyclopropyl-N-(4-fluoro-3-methyl-phenyl)-3-hydroxy-thioacrylamide (i.e. HK1), 2-Cyano-3-cyclopropyl-3-hydroxy-N-(3-methyl-4-nitro-phenyl)-thioacrylamide (i.e. HK3), a tautomer of HK1, a tautomer of HK3, or mixtures thereof, or pharmaceutically acceptable salts thereof or solvates thereof,
wherein the subject is in a caloric surplus state.

**10.** Cosmetic, non-therapeutic use according to claim 9, wherein the use comprises administering at least 5 mg/kg per body weight of the subject, preferably at least 7.5 mg/kg per body weight of the subject, preferably at least 10 mg/kg per body weight of the subject, of the thioacrylamide derivative.

Fig. 1

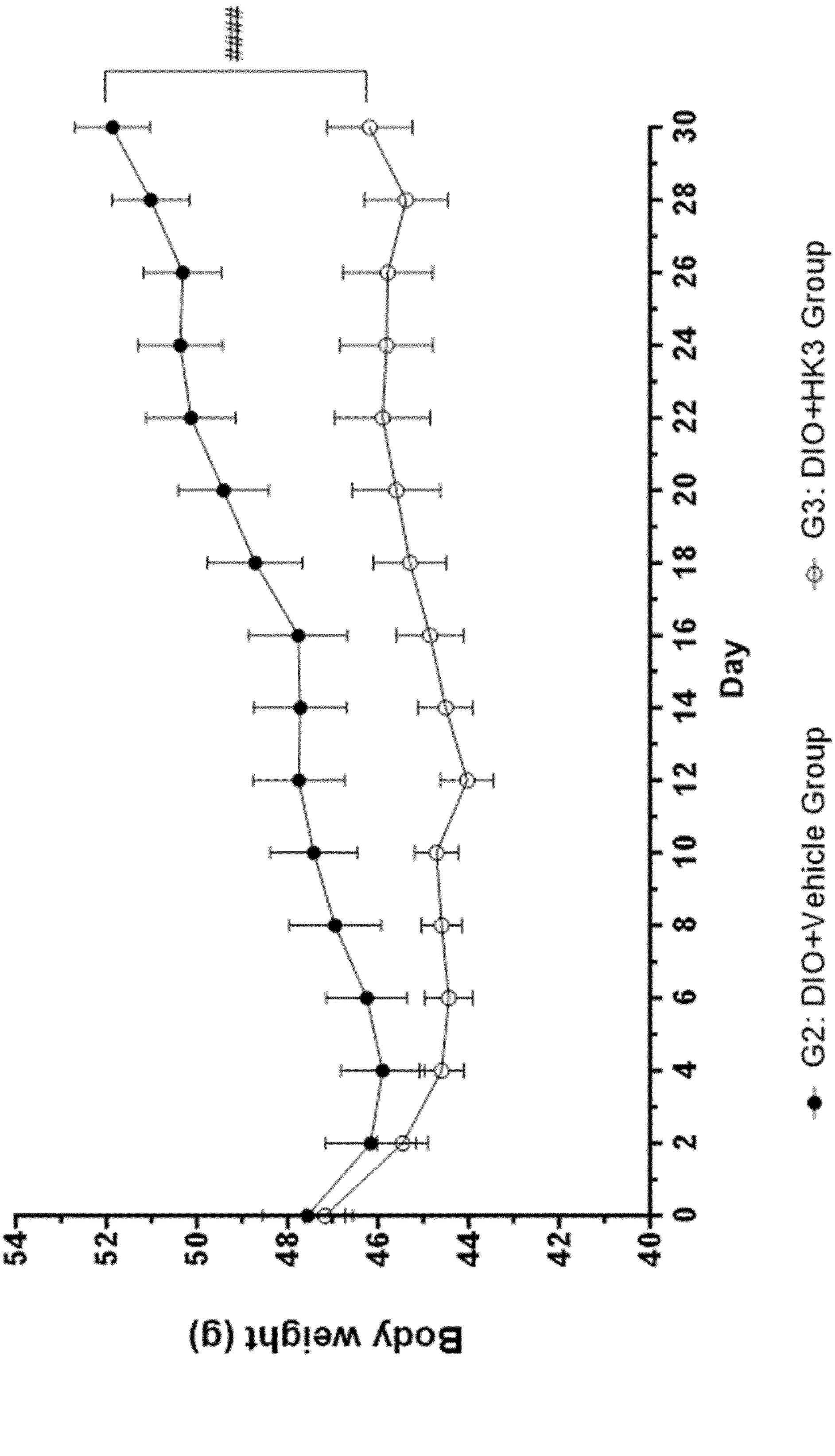

####
Body weight (g)
54
52
50
48
46
44
42
40
Day
0
2
4
6
8
10
12
14
16
18
20
22
24
26
28
30
G2: DIO+Vehicle Group
G3: DIO+HK3 Group

Fig. 2

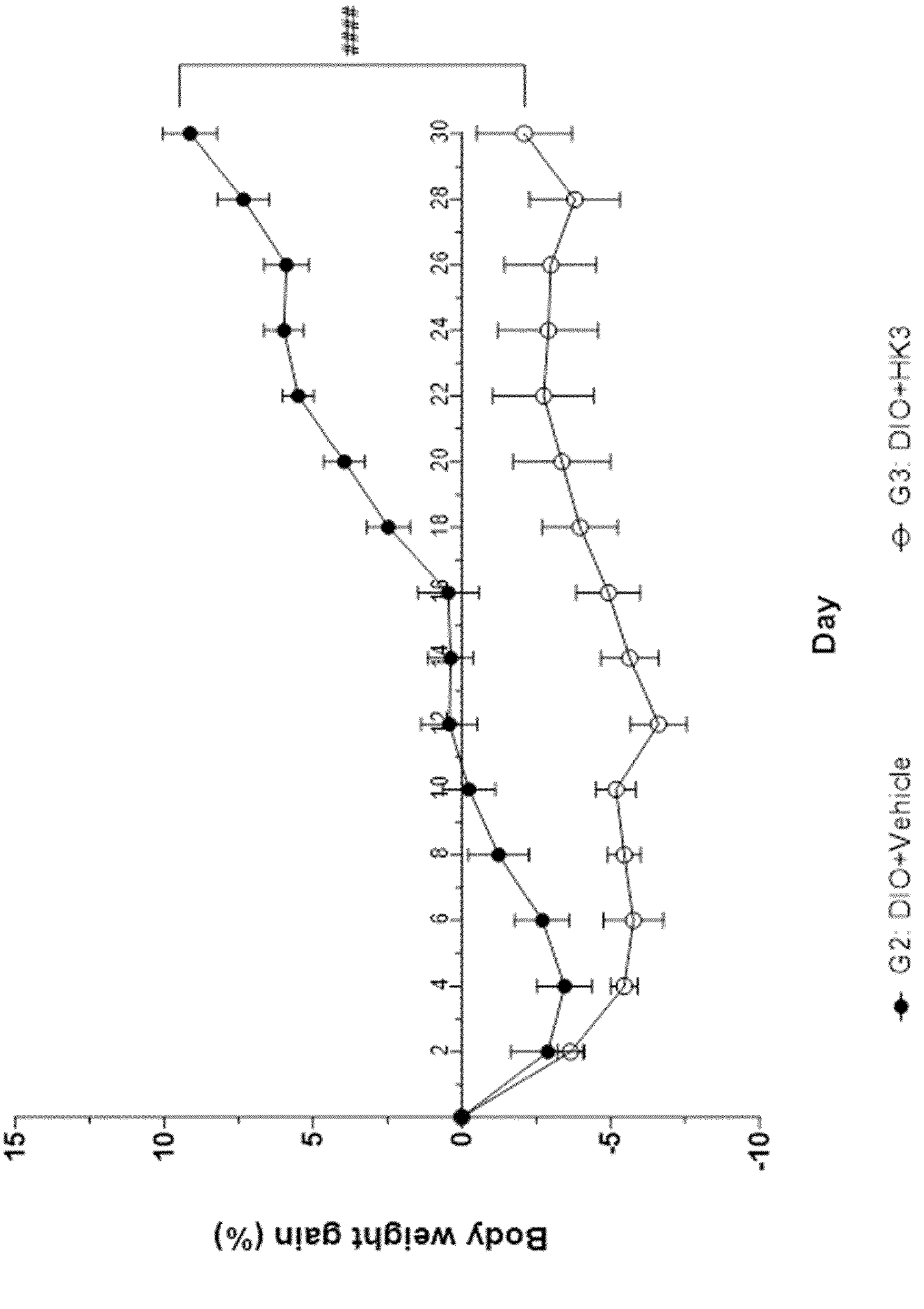
####
Body weight gain (%)
15
10
5
0
-5
-10
Day
2
4
6
8
10
12
14
16
18
20
22
24
26
28
30
G2: DIO+Vehicle
G3: DIO+HK3

Fig. 3

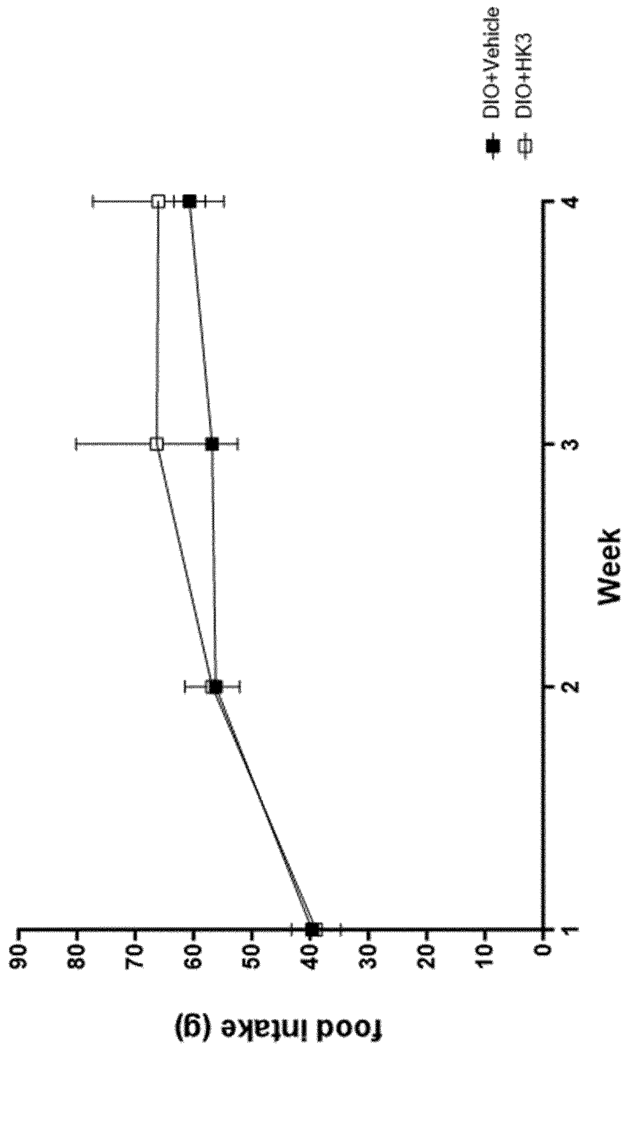
food intake (g)
90
80
70
60
50
40
30
20
10
0
1
2
3
4
Week
DIO+Vehicle
DIO+HK3

Fig. 4

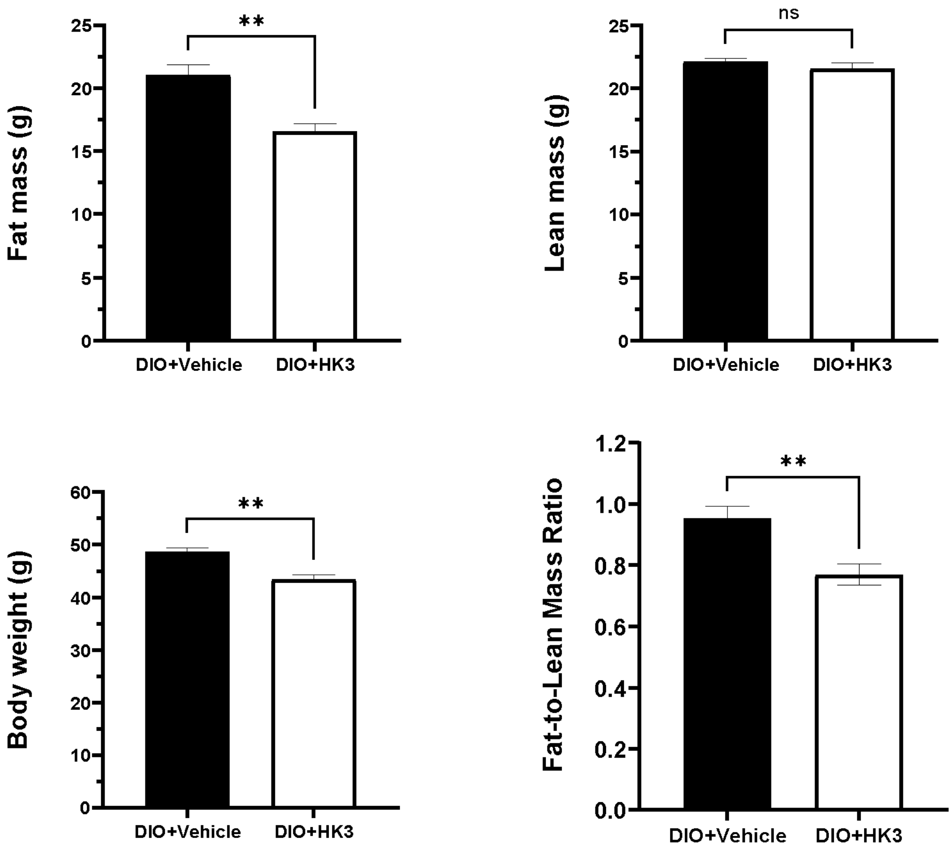
Fat mass (g)
25
20
15
10
5
0
**
DIO+Vehicle
DIO+HK3
Lean mass (g)
ns
DIO+Vehicle
DIO+HK3
Body weight (g)
60
50
40
30
20
10
0
**
DIO+Vehicle
DIO+HK3
Fat-to-Lean Mass Ratio
1.2
1.0
0.8
0.6
0.4
0.2
0.0
**
DIO+Vehicle
DIO+HK3

Fig. 5

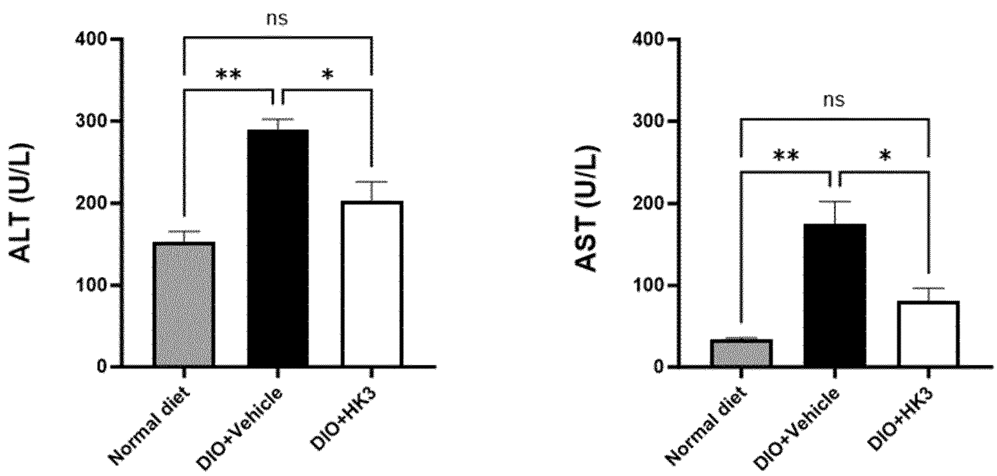
ALT (U/L)
400
300
200
100
0
ns
**
*
Normal diet
DIO+Vehicle
DIO+HK3
AST (U/L)
ns
**
*
Normal diet
DIO+Vehicle
DIO+HK3

Fig. 6

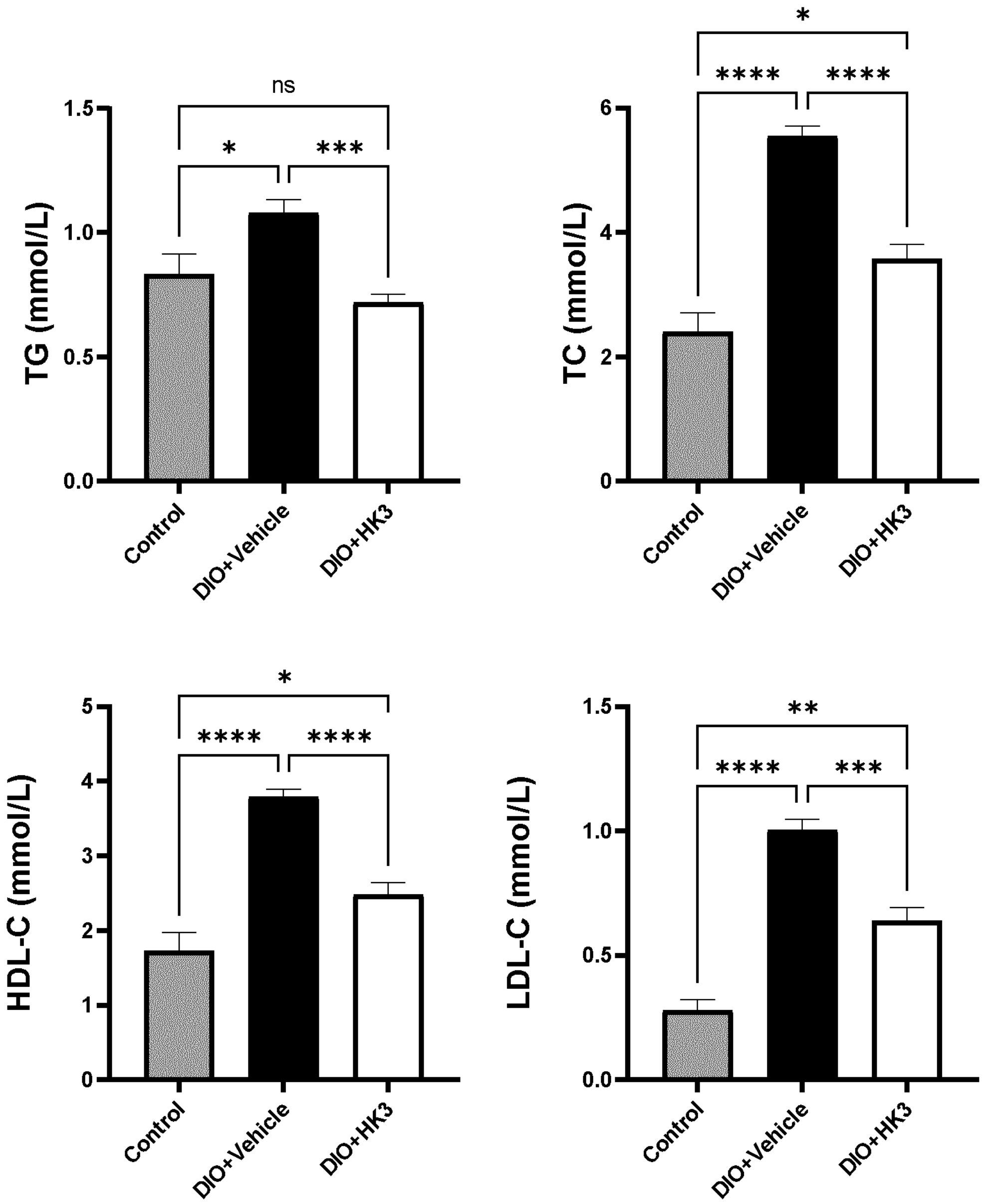

ns
*
***
1.5
1.0
0.5
0.0
TG (mmol/L)
Control
DIO+Vehicle
DIO+HK3
*
****
****
6
4
2
0
TC (mmol/L)
Control
DIO+Vehicle
DIO+HK3
*
****
****
5
4
3
2
1
0
HDL-C (mmol/L)
Control
DIO+Vehicle
DIO+HK3
**
****
***
1.5
1.0
0.5
0.0
LDL-C (mmol/L)
Control
DIO+Vehicle
DIO+HK3

Europäisches Patentamt
European Patent Office
Office européen des brevets

# EUROPEAN SEARCH REPORT

Application Number
EP 24 20 9123

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2020/384086 A1 (BIRNIR BRYNDIS [SE] ET AL) 10 December 2020 (2020-12-10)<br>* claims 1,14 *<br>* paragraph [0001] - paragraph [0008] *<br>* paragraph [0029] *<br>* paragraph [0057] *<br>----- | 1,2,4-7,9<br>1-10 | INV.<br>A61K31/277<br>A61P3/04 |
| Y | US 2017/088513 A1 (HASSE BIRGIT [DE] ET AL) 30 March 2017 (2017-03-30)<br>* paragraphs [0001] - [0008], [0029], [0057] *<br>* claims 1, 14 *<br>----- | 1-10 | |
| Y | KR 2021 0157892 A (KIM BYEONG HA [KR]; KIM DONG WOOK [KR]; KANG KI SUNG [KR])<br>29 December 2021 (2021-12-29)<br>* claim 1 *<br>----- | 1-10 | |

TECHNICAL FIELDS SEARCHED (IPC)

A61K
A61P

The present search report has been drawn up for all claims

1

| Place of search | Date of completion of the search | Examiner |
|---|---|---|
| Munich | 18 March 2025 | Olausson Boulois, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 24 20 9123

This annex lists the patent family members relating to the patent documents cited in the above-mentioned European search report. The members are as contained in the European Patent Office EDP file on
The European Patent Office is in no way liable for these particulars which are merely given for the purpose of information.

18-03-2025

| Patent document cited in search report | Publication date | Patent family member(s) | Publication date |
|---|---|---|---|
| US 2020384086 A1 | 10-12-2020 | EP 3755314 A1 | 30-12-2020 |
| | | US 2020384086 A1 | 10-12-2020 |
| | | WO 2019162403 A1 | 29-08-2019 |
| US 2017088513 A1 | 30-03-2017 | AU 2015233686 A1 | 22-09-2016 |
| | | CA 2941782 A1 | 24-09-2015 |
| | | CN 106458878 A | 22-02-2017 |
| | | EP 3119744 A1 | 25-01-2017 |
| | | JP 6488000 B2 | 20-03-2019 |
| | | JP 2017510632 A | 13-04-2017 |
| | | PL 3119744 T3 | 31-07-2019 |
| | | US 2017088513 A1 | 30-03-2017 |
| | | WO 2015140081 A1 | 24-09-2015 |
| KR 20210157892 A | 29-12-2021 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2024114970 A1


### Non-patent literature cited in the description


- **POTTER et al.** Defining Overweight and Obesity by Percent Body Fat instead of Body Mass Index. *J Clin Endocrinol Metab*, 15 May 2024
- **NEELAND et al.** Changes in lean body mass with glucagon-like peptide-1-based therapies and mitigation strategies.. *Diabetes, obesity & metabolism*, 2024, vol. 26 (4), 16-27